# EUROPEAN PATENT APPLICATION

(11) **EP 4 498 384 A1**
(43) Date of publication of application: **29.01.2025**
(21) Application number: 23188445.3
(22) Date of filing: 28.07.2023
(51) Int. Cl.: G16H 40/60, G16H 40/63, G06F 3/01, G06F 40/10, G06F 40/30, G06Q 10/10, G09B 5/00, G09B 5/06, G10L 15/26

(54) **COMMUNICATION SYSTEM FOR UPDATING LECTURE TEXT USING SLIDE SPECIFIC USER DATA**

(71) Applicant: Koninklijke Philips N.V., 5656 AG Eindhoven (NL)
(72) Inventor: ANAND, Shreya, Eindhoven (NL); VAN EE, Raymond, Eindhoven (NL); JELFS, Sam Martin, 5656AG Eindhoven (NL); MENDOZA, Jose Luis Diaz, Eindhoven (NL); RUTJES, Heleen, Eindhoven (NL); WESTERINK, Joanne Henriëtte Desirée Monique, Eindhoven (NL)
(74) Representative: Philips Intellectual Property & Standards

(57) **Abstract**

Disclosed herein is a medical system (100, 300) comprising a communication system (110) configured for establishing a teleconference session between multiple telecommunication devices (106). The medical system further comprises a memory (116) storing machine executable instructions (120), at least one presentation slide (122), and presentation slide specific lecture text (124). The medical system further comprises a computational system (110). Execution of the machine executable instructions causes the computational system to repeatedly: receive (200) presentation slide specific user data (128) from the communications system; construct (202) presentation slide specific modification text (126) using the presentation slide specific user data; and modify (204) the presentation slide specific lecture text using the presentation slide specific modification text.

## Description

### TECHNICAL FIELD

The invention relates to medical communications and telemedicine.

### BACKGROUND

Medical communications may involve a number or participants each sharing audio and or visual data from different sources. For example, participants may share medical images to discuss cases. In other examples participants may participate in the examination or diagnosis of a clinical subject. In other examples participants may receive training on how to use a medical imaging system. Images of the medical imaging system, as well as user interfaces may be reproduced for participants.

### SUMMARY

The invention provides for a medical system, a method, and a computer program in the independent claims. Embodiments are given in the dependent claims.

In one aspect the invention provides for a medical system that comprises a communication system that is configured for establishing a teleconference session between multiple telecommunication devices. The medical system further comprises a memory storing machine-executable instructions. The memory further stores at least one presentation slide. The memory further stores presentation slide-specific lecture text.

The medical system further comprises a computational system. Execution of the machine-executable instructions causes the computational system to repeatedly receive presentation slide-specific user data from the communication system. Execution of the machine-executable instructions further causes the computational system to repeatedly construct presentation slide-specific modification text using the presentation slide-specific user data. Execution of the machine-executable instructions further causes the computational system to repeatedly modify the presentation slide-specific lecture text using the presentation slide-specific modification text.

In another aspect the invention provides for a method of operating the medical system. The medical system comprises a communication system configured for establishing a teleconference session between multiple telecommunication devices. The medical system further comprises a memory storing at least one presentation slide and presentation slide-specific lecture text. The method comprises repeatedly receiving presentation slide-specific user data from the communication system. The method further comprises repeatedly constructing presentation slide-specific modification text using the presentation slide-specific user data. The method further comprises repeatedly modifying the presentation slide-specific lecture text using the presentation slide-specific modification text.

In another aspect the invention provides for a computer program comprising machine-executable instructions for execution by a computational system controlling the medical system. The medical system comprises a communication system configured for establishing a teleconference session between multiple telecommunication devices. The medical system further comprises a memory storing at least one presentation slide and presentation slide-specific lecture text. Execution of the machine-executable instructions causes the computational system to repeatedly receive presentation slide-specific user data from the communication system. Execution of the machine-executable instructions further causes the computational system to repeatedly construct presentation slide-specific modification text using the presentation slide-specific user data. Execution of the machine-executable instructions further causes the computational system to repeatedly modify the presentation slide-specific lecture text using the presentation slide-specific modification text.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig 1.
Fig. 3 illustrates a further example of a medical system.
Fig. 4 shows a flow chart which illustrates a method of using the medical system of Fig 3.

### DESCRIPTION OF EMBODIMENTS

Like numbered elements in these Figures are either equivalent elements or perform the same function. Elements which have been discussed previously will not necessarily be discussed in later Figures if the function is equivalent.

Examples may be beneficial because they may provide for a means of automatically updating or improving the presentation slide-specific lecture text using the presentation slide specific user data that may collected from the telecommunication devices of individual users.

A teleconference session as used herein encompasses a communication session which includes audio and/or visual data being exchanged between participants. Various types of telecommunication devices may be used for a teleconference session. For example, various portable computing devices such as a smartphone or a laptop may be used as well as a computer or even a dedicated telecommunication device for teleconference sessions.

A presentation slide as used herein encompasses visual data which may be presented via the teleconference session. During presentations often times individual slides are used to provide graphics or other visual data to participants. The presentation slide-specific lecture text may encompass lecture text and/or talking points descriptive of the at least one presentation slide. The presentation slide-specific lecture text may for example be arranged and provide various text and talking points for each individual presentation slide.

The presentation slide-specific user data may for example encompass data descriptive of or received from individual users and are grouped according to individual presentation slides.

In another example the memory further stores an audio-to-text conversion module configured to generate text in response to inputting an audio stream comprising speech. The audio-to-text conversion module may for example be implemented using a specialized neural network or commercially available audio to text conversion program or application.

Execution of the machine-executable instructions further causes the computational system to receive a question audio stream from one of the multiple telecommunication devices. Execution of the machine-executable instructions further causes the computational system to receive a question text in response to inputting the question audio stream into the audio-to-text conversion module. Execution of the machine-executable instructions further causes the computational system to receive the presentation slide-specific modification text using the question text. This example may be beneficial because it provides a means of modifying the presentation slide-specific lecture text using questions which are received from participants.

An example like this may enable the questions of the students or other participants to be analyzed (or at least the answers that the teacher gives to these questions). This may be an indication of which topics/elements are considered difficult by the students. These topics might benefit from an update in their presentation. A presentation slide specific lecture text (or baseline) could be created offline in which the teachers are prompted to spend more time on that particular topic. Then the alternative baseline is used to guide the teachers in the upcoming sessions. After N of these alternative-baseline-based sessions it is evaluated whether the alternative baseline indeed performs better, that is: whether it generates a lower number of questions on the difficult topic involved. If so, the alternative baseline is retained for future use.

In another example the audio-to-text conversion module is further configured to output one or more speech metric in response to receiving the audio stream. Execution of the machine-executable instructions further causes the computational system to receive the one or more speech metric in response to inputting the user specific audio stream into the audio-to-text conversion module. Execution of the machine-executable instructions further causes the computational system to enable or disable modification of the presentation slide-specific lecture text using the one or more speech metric. This example may be beneficial because it may provide a means of determining if a question should or should not be used to modify the presentation slide-specific lecture text.

In another example the one or more speech metric comprises a measured reaction time to a question in the audio stream. This example may be beneficial because it may be useful in measuring an attentiveness or attention that an applicant is paying to a particular question. It may also be used as a measure if the subject understands the question.

In another example the one or more speech metric further comprises a pitch range or change in the pitch range of the subject speech in the audio stream. Noting changes in the pitch range for a particular subject may be useful because they may indicate if a question should be used to modify the presentation slide-specific lecture text or not.

In another example the one or more speech metric further comprises a volume range or change in the volume range of the speech text in the audio stream. This example may also be beneficial because if a person is speaking with more dynamic range or in a monotone it may indicate the level of the interest and the importance of including text generated from a question into the presentation slide-specific lecture text.

In another example the speech metric comprises an indication of the language of the subject speech in the audio stream. For example, a change in the language that is different from that being presented may also indicate a breakdown in communication.

In another example an emotional state is estimated using a natural language processing and/or sentiment analysis of the audio stream. This may be useful as it may provide a measure or means to determine how frustrated an individual is with a particular presentation. If a person is more animated or frustrated it may be more beneficial to include more content in the presentation slide-specific lecture text.

In another example the one or more speech metric further comprises a syntactic analysis comparing the question text with the text generated from the audio stream of the multiple communication devices to detect repeated questions or interruptions of speech. The detection of the repeated questions or interruptions of speech may for example be beneficial in determining information which is particularly important to include in the presentation slide-specific lecture text.

In another example the presentation slide-specific user data comprises a question text. Execution of the machine-executable instructions further causes the computational system to determine the presentation slide-specific modification text using the question text. In this example the text of the question is used to provide the content for modifying the presentation slide-specific lecture text. This may be beneficial as it ensures that the contents of the question text are included in further presentations.

In another example execution of the machine-executable instructions further causes the computational system to display the question text on a user interface. Execution of the machine-executable instructions further causes the computational system to receive the presentation slide-specific modification text from the user interface in response to displaying the question text. In this example the text used for modifying the presentation slide-specific lecture text is received in response to displaying it on a user interface. This may provide a convenient means for a user to provide this information.

In another example the memory further stores a text generating large language model that is configured to generate the presentation slide-specific modification text in response to receiving the question text and preferably receiving the presentation slide-specific lecture text. Execution of the machine-executable instructions further causes the computational system to receive the presentation slide-specific modification text in response to inputting the question text and preferably the presentation slide-specific lecture text into a text generating large language model. This example may be beneficial because it provides a means of automatically generating the presentation slide-specific modification text. It may for example be used to take into account the question as well as the existing text in the presentation slide-specific lecture text. This may therefore provide a very efficient and automated way of updating the presentation slide-specific lecture text.

A large language model (LLM) as used herein encompasses a neural network architecture, typically built up of transformers (encoders and decoders) with self-attention layers and residual connections, that is a language model that has been trained using unlabeled text using self-supervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

In this example the text generating large language model is a large language model that has been trained or configured (using fine tuning) to generate the presentation slide-specific modification text in response to receiving the question text and preferably receiving the presentation slide-specific lecture text.

In another example the presentation slide-specific user data comprises physiological data. Execution of the machine-executable instructions further causes the computational system to enable or disable modification of the presentation slide-specific lecture text using the physiological data. This example may be beneficial because it may provide for a means of enabling or disabling modification of the slide-specific lecture text using involuntary reaction data of the subject.

In another example the user-specific physiological data comprises sensor data collection by the multiple telecommunication devices from user-specific wearable devices. This for example may include heart rate sensors, skin connectivity sensors and other types of sensors.

In another example the memory further comprises an image recognition module that is configured to output one or more image metrics in response to receiving a video stream. The presentation slide-specific user data comprises the video stream comprising user-specific images collected by the multiple telecommunication devices. Execution of the machine-executable instructions further causes the computational system to receive the one or more image metric in response to inputting the image stream into the image recognition module. Execution of the machine-executable instructions further causes the computational system to enable or disable modification of the presentation slide-specific lecture text using the one or more image metric. This may be beneficial because it may provide for a means of automatically enabling or disabling the modification of the presentation slide-specific lecture text using images of participants that are normally available during a teleconferencing session.

The image recognition module could for example be an image processing neural network that is configured to output the one or more image metric in response to receiving the video stream.

In another example the one or more image metric comprises a facial score that is assigned using facial expression analysis.

In another example the one or more image metric comprises a gesture score assigned using gesture analysis and/or a frequency of gestures using gesture analysis. For example, the gesture score could be comprised by using a neural network that automatically identifies joint locations of the subject and then by classifying the gestures according to the positions of the subject's joints or appendages.

In another example the image metric comprises a gaze analysis score correlated to time viewing the device-specific rendering. For example, by using a camera it could be determined if the subject is looking at the presentation or is distracted and looking somewhere else.

In another example the one or more image metric may comprise a heart rate. For example if a color image of a subject is used small changes in the face or other exposed skin patches may be used to monitor the heart rate.

In another example the one or more image metric comprises a breathing rate. Similarly, motion of the subject's chest region may be used for monitoring the breathing rate.

In another example the memory further stores an audio conversion module configured to output presenter text in response to a presenter audio stream. The memory further comprises a text comparison module that is configured for outputting a textual difference between two input texts. Execution of the machine-executable instructions further causes the computational system to repeatedly receive a presenter audio stream from the communication system. Execution of the machine-executable instructions further causes the computational system to repeatedly receive presenter text in response to inputting the presenter audio stream into the audio conversion module. The audio conversion module may be identical with the audio-to-text conversion module mentioned above. In other examples they are separate modules.

Execution of the machine-executable instructions further causes the computational system to repeatedly receive the textual difference in response to inputting the presenter text and the slide-specific lecture text of the one or the at least one presentation slide as a two input text into the text comparison module. Execution of the machine-executable instructions further causes the computational system to repeatedly provide a signal to a presenter user interface if the textual difference is received. This may for example be used to warn a presenter that a certain topic has not been presented or it may be used to further control the operation of the communication system.

Execution of the machine-executable instructions further causes the computational system to display a missing text message descriptive of the textual difference on the presenter user interface if the signal indicates that the presenter text is missing a portion of the presentation slide-specific lecture text. This may be beneficial because it may provide a means of prompting the presenter to present a particular topic automatically.

In another example execution of the machine-executable instructions further causes the computational system to display a modified lecture text message descriptive of the textual difference on the presenter user interface if the signal indicates that the slide-specific text is missing a portion of the presenter text. Execution of the machine-executable instructions further causes the computational system to receive a positive modification selection or a negative modification selection from the presenter user interface in response to displaying the modified lecture text message on the presenter user interface. Execution of the machine-executable instructions further causes the computational system to add the missing portion of the presenter text to the slide-specific text if the positive modification selection is received. This may be beneficial as it may provide for a manual means of automatically adding text to the presentation slide-specific lecture text.

In another example the text comparison module is implemented using natural language processing. The natural language processing preferably comprises term frequency-inverse document frequency processing.

In another example the text comparison module is implemented using a text comparison large language model. For example, the text comparison large language model could be a large language model that has been configured to compare two input texts and to provide a difference between the two. This may for example be able to provide a comparison of the content of the two texts as opposed to a straight character comparison as would be done by comparing text files directly.

In this example the text comparison large language model is a large language model that has been trained or configured (using fine tuning) to compare two input texts and to provide a difference between the two.

In another example the memory further stores multiple sets of slide-specific lecture text. Execution of the machine-executable instructions further causes the computational system to receive a set selection descriptive of one set of the multiple sets of slide-specific lecture texts. Execution of the machine-executable instructions further causes the computational system to choose the slide-specific lecture text using the set selection. This may be used to provide a particular group of presentation slide-specific lecture texts. This may for example be useful in presenting the same set of slides to different groups or audiences.

Below are a number of example scenarios where there is a set selection of one of the multiple sets of slide-specific lecture texts:
1) A python training course can be divided into three levels beginner, intermediate and advanced course. The set selection would be for beginner, intermediate, or advanced. The training material for these 3 courses is different apart from some overlapping material. There might be scenarios that one trainer delivers 3 courses at different time intervals in a day, or a different trainer can deliver the course on another day. The audience for both the scenarios are different. Due to limitations of a human, the trainer taking up the training sessions of basic, intermediate and advance on the same day can miss out on providing a crucial part of information in any of the sessions. The way of teaching and the quality and quantity of information can vary from trainer to trainer. Having a baseline to check the lectures delivered in real-time can aid the trainer by prompting the piece of information that was relevant to that session.
2) A second scenario is about one trainer delivering lectures/messages to a different set of audiences, for example trainees having a medical background, physics background and mathematics background. This provides three different set selections. The content varies widely based on the interest of the audience. Having a baseline created for each set of trainees helps in pulling out relevant information from a prestored slide deck that eases the work of the trainer as well as helps in the smooth flow of information during the sessions without having to compromise on the quality of b information provided by the trainer and catering to the interest of the trainees.

This can also be extended to a situation where a doctor/healthcare specialist is delivering a training session to a different set of students. E.g., novice and/or advanced. The content of the session and the level of teaching varies drastically in the two session based on the understanding/grasping level of the students. Having a pre formulated set of content slides for each of the sessions helps the doctor focus on the content rather than tailoring the content according to the needs of the session.

In another example the signal is provided during further reception of the presenter audio stream. This may for example provide for modifications of the presentation in real time or provide a warning that a topic has been missed in real time.

For example, NLP (Natural Language Processing) processing using a large language module may be in real-time during the training, and feedback is given directly to the trainer during the session. By comparing the training being given (as contained in the presenter audio stream) against the presentation slide specific lecture text in could possibly be ensured that baseline key points that might have been missed can be highlighted, and the trainer can ensure that they are covered before the end of the session.

In another example execution of the machine-executable instructions further causes the computational system to receive a baseline audio stream. Execution of the machine-executable instructions further causes the computational system to receive baseline text in response to inputting the baseline audio stream into the audio-to-text conversion module. Execution of the machine-executable instructions further causes the computational system to construct the slide-specific lecture text using the baseline text. This may for example provide a means of automatically constructing the presentation slide-specific lecture text.

In another example execution of the machine-executable instructions further causes the computational system to generate handout pages using the at least one presentation slide and the slide-specific lecture text. Execution of the machine-executable instructions further causes the computational system to transmit the handout pages to the multiple telecommunication devices using the communication system. This may provide for a convenient means of distributing the handout pages to participants.

In another example the medical system further comprises a medical imaging system. The at least one presentation slide comprises acquired medical images and/or a control user interface rendering. This may be beneficial as it may provide for an improved means of sharing the medical imaging results and/or training people on the use of the medical imaging system.

In another example the medical system further comprises a medical image database. The at least one presentation slide at least partially comprises data retrieved from the medical image database. This may for example be useful in situations where particular medical images or information are being discussed as a group.

In another example execution of the machine-executable instructions further causes the computational system to transmit one of the at least one presentation slide and a presenter audio stream to the multiple telecommunication devices using the communication system.

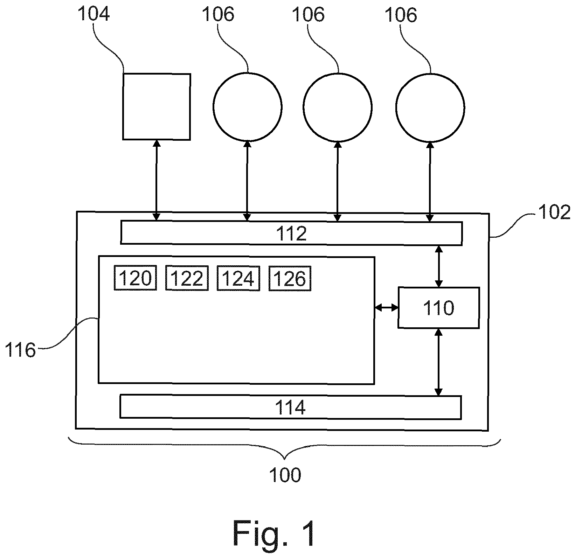

Fig. 1 illustrates an example of a medical system 100. The medical system 100 is shown as comprising a computer 102. There is also an optional medical image source 104. The medical image source 104 could for example be a medical imaging system. The medical imaging system may provide presentation slides that comprise acquired medical images and/or a control user interface of the medical imaging system. The medical imaging system may for example be a computed tomography system, a positron emission tomography system, a single-photon emission tomography system, a digital X-ray, a digital fluoroscope, or a magnetic resonance imaging system. In other examples, the medical image source 104 may be a medical image database that retrieves prior acquired medical images.

A number of telecommunication devices 106 are also shown. A telecommunication device 106 may for example be a smartphone, a computer, a laptop, a tablet computer or other computing device.

The computer 102 is shown as comprising a computational system 110 that is in communication with a communication system 112. In this case the communication system 112 may be a network interface. The computational system 110 is further shown as being in communication with a user interface 114 and a memory 116. The user interface 114 may for example enable an operator of the computer 102 to provide video and/or audio data during a teleconferencing session. The user interface 114 may also enable the operator to control the operation and function of the medical system 100. The memory 116 is intended to represent various types of memories which are accessible to the computational system 110. In some examples the memory 116 may comprise a non-transitory storage medium.

The memory 116 is shown as containing machine-executable instructions 120. The machine-executable instructions 120 for example enable the computational system 110 to use the network interface 112 to establish a teleconferencing session between the multiple telecommunication devices 106 and the computer 102. The memory 116 is shown as containing at least one presentation slide 122. The memory 116 is further shown as containing presentation slide-specific lecture text 124 for the particular presentation slide 122. In case there are multiple presentation slides 122 there may be multiple presentation slide-specific lecture texts 124 that fit that respective presentation slides. In some examples there may be multiple groups of presentation slide-specific lecture texts 124 to enable an operator to change the lecture text by selecting one of the multiple groups. The memory 116 is further shown as containing slide-specific modification text 126. The slide-specific modification text 126 is text which may be used to add to or modify the presentation slide-specific lecture text 124.

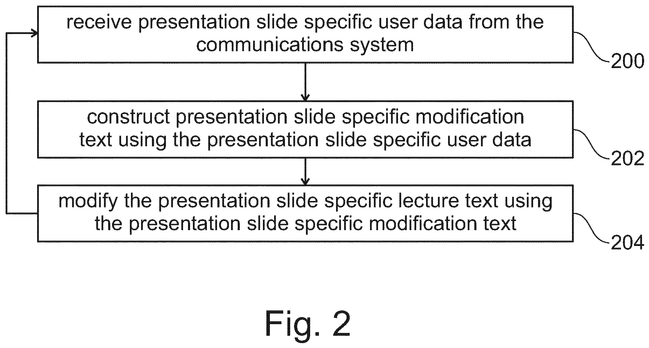

Fig. 2 shows a flowchart which illustrates a method of operating the medical system 100 of Fig. 1. In step 200, presentation slide-specific user data is received. The presentation slide-specific user data is received from the communication system 112 from the various telecommunication devices 106. This may include information that is received during the presentation of a particular presentation slide 122. This may include such things as audio or visual data provided by a particular telecommunication device 106 for a particular user. It may also in some instances include such things as logical data that was recorded by a particular telecommunication device 106. For example, the telecommunication device 106 may acquire physiological data from a particular user or it may also connect to a sensor which is measuring physiological data from a subject. For example, a telecommunication device 106 may communicate with a wearable device such as a smartwatch worn by a user. In step 202, the presentation slide-specific modification text 126 is constructed using the presentation slide-specific user data and is used to modify the presentation slide-specific modification text 126. For example, audio data of the subject could be converted into questions which are then used to provide additional answers or text to the slide-specific user data 128.

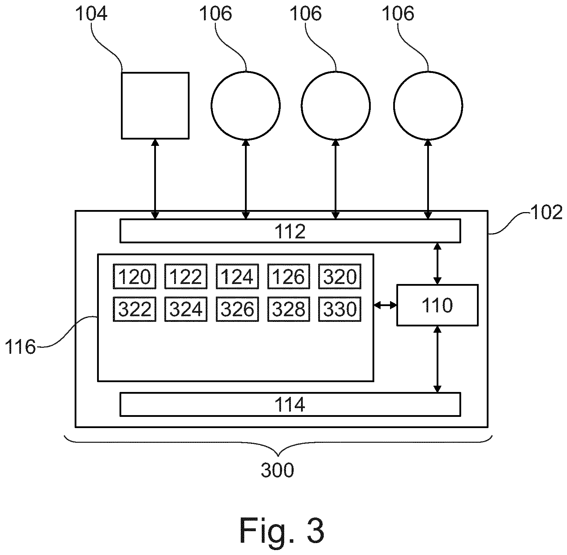

Fig. 3 illustrates a further example of a medical system 300. The medical system 300 is similar to the medical system 100 depicted in Fig. 1 with additional software components stored in the memory 116.

The memory 116 is further shown as containing an audio-to-text conversion module 320. The memory 116 is further shown as containing a question audio stream 322 that was received from a participant using one of the telecommunication devices 106. The questions audio stream 322 may be received as part of the presentation slide-specific user data128. The memory 116 is further shown as containing a question text 324 that is received by inputting the question audio stream 322 into the audio-to-text conversion module 320. The question text 324 may for example represent a question asked by a user of one of the telecommunication devices 106 when the presentation slide 122 was presented. This for example may represent information or data which was not known to the user of the telecommunication device 106. The memory 116 is further shown as containing a text generating large language module 326. The text generating large language module 326 is configured to receive at least the question text 324 and also possibly the presentation slide-specific lecture text 124 and output the slide-specific modification text 126 in response.

The memory 116 is further shown as containing physiological data 328 which may have been received by the same telecommunication device 106 that provided the question audio stream 322. The physiological data 328 may include such things as audio data, video data, or sensor data descriptive of the subject's physiological condition. Depending upon the type of physiological data 328, a decision module 330 is used to enable or disable modification of the presentation slide-specific lecture text 124 using the slide-specific modification text 126.

In this example, the presentation slide-specific user data provides both the question audio stream and the physiological data from a particular user or participant. The question audio stream and the physiological data may be correlated. This may have the advantage that the presentation slide-specific user data 128 is used for both generating the slide specific modification text and controlling whether the slide specific modification text is to be used to update the presentation slide specific lecture text or not. The physiological data may provide a means of determining objectively if e.g., the user or participant had a difficult time with understanding the presentation slide or if the participant were simply asking a question to be polite or feign interest. For example, if the physiological data 328 comprised eye motion data it could be determined if the user was actually paying attention to the presentation slide 122 or not. In this example, in case the attention of the user, as indicated by physiological data, could be used to determine if the subject's question was due to a lack of explanation or due to a lack of attention or interest on the part of the subject.

Similarly, a change in the user's blood pressure or skin conductivity could indicate stress or trouble in understanding the presentation slide 122. In this example, if the blood pressure or skin conductivity were used as a measure of stress it may be a good measure if the explanation for a presentation slide is complete or not. For example, if the user's blood pressure suddenly rises and then the user asks a question, it could indicate that the question is very relevant.

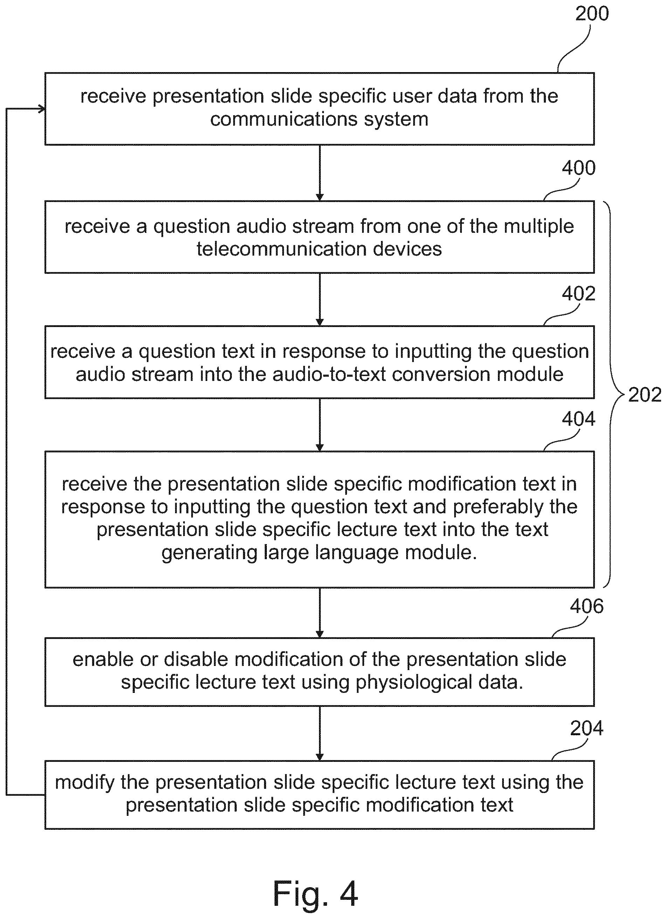

Fig. 4 shows a flowchart which represents a method of operating the medical system 300 of Fig. 3. The method illustrated in Fig. 4 is similar to the method illustrated in Fig. 2. Step 200 is performed as was illustrated in Fig. 2. In this example, steps 400, 402, 404 are equivalent to step 202 in Fig. 2. In step 400 the question audio stream 322 is received from one of the multiple telecommunication devices 106. In step 402, the question text 324 is received in response to inputting the question audio stream 322 into the audio-to-text conversion module 320. In step 404, the presentation slide-specific modification text 126 is received in response to inputting the question text and preferably the presentation slide-specific lecture text into the text generating large language model 326. The physiological data 328 received from the same telecommunication device 106 as the question audio stream 322 is used to enable or disable modification of the presentation slide-specific lecture text 124 using the slide-specific modification text 126. For example, the physiological data 328 may be input into the decision module 330 which may contain a set of predetermined criterion or qualities to decide if the presentation slide-specific lecture text 124 should be modified or not.

It is understood that one or more of the aforementioned examples or embodiments of the invention may be combined as long as the combined examples or embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

100 medical system
102 computer
104 medical image source
106 telecommunication device
110 computational system
112 communication system (network interface)
114 user interface
116 memory
120 machine executable instructions
122 presentation slide
124 presentation slide specific lecture text
126 slide specific modification text
128 presentation slide specific user data
200 receive presentation slide specific user data from the communications system
202 construct presentation slide specific modification text using the presentation slide specific user data
204 modify the presentation slide specific lecture text using the presentation slide specific modification text
300 medical system
320 audio-to-text conversion module
322 question audio stream
324 question text
326 text generating large language model
328 physiological data
330 decision module
400 receive a question audio stream from one of the multiple telecommunication devices
402 receive a question text in response to inputting the question audio stream into the audio-to-text conversion module
404 receive the presentation slide specific modification text in response to inputting the question text and preferably the presentation slide specific lecture text into the text generating large language module
406 enable or disable modification of the presentation slide specific lecture text using the physiological data

## Claims

1. A medical system (100, 300) comprising:
- a communication system (110) configured for establishing a teleconference session between multiple telecommunication devices (106);
- a memory (116) storing machine executable instructions (120), at least one presentation slide (122), and presentation slide specific lecture text (124);
- a computational system (110), wherein execution of the machine executable instructions causes the computational system to repeatedly:
- receive (200) presentation slide specific user data (128) from the communications system;
- construct (202) presentation slide specific modification text (126) using the presentation slide specific user data; and
- modify (204) the presentation slide specific lecture text using the presentation slide specific modification text.

2. The medical system of claim 1, wherein the memory further stores an audio-to-text conversion module (320) configured to generate text in response to inputting an audio stream comprising speech, wherein execution of the machine executable instructions further causes the computational system to:
- receive (400) a question audio stream (322) from one of the multiple telecommunication devices;
- receive (402) a question text (324) in response to inputting the question audio stream into the audio-to-text conversion module; and
- receive the presentation slide specific modification text using the question text.

3. The medical system of claim 2, wherein the audio-to-text conversion module is further configured to output one or more speech metric in response to receiving the audio stream, wherein execution of the machine executable instructions further causes the computational system to receive the one or more speech metric in response to inputting the user specific audio stream into the audio-to-text conversion module, wherein execution of the machine executable instructions further causes the computational system to enable or disable modification of the presentation slide specific lecture text using the one or more speech metric.

4. The medical system of claim 2 or 3, wherein the audio-to-text conversion module is further configured to output presenter text in response to inputting a presenter audio stream, wherein the memory further comprises a text comparison module configured for outputting a textual difference between two input texts;
- a computational system, wherein execution of the machine executable instructions causes the computational system to repeatedly:
- receive a presenter audio stream from the communication system;
- receiving presenter text in response to inputting the presenter audio stream into the audio-to-text conversion module;
- receiving the textual difference in response to inputting the presenter text and the slide specific lecture text of the one of the at least one presentation slide as the two input texts into the text comparison module; and
- provide a signal to a presenter user interface if the textual difference is received.

5. The medical system of claim 4, wherein execution of the machine executable instructions further causes the computational system to:
- display a modify lecture text message descriptive of the textual difference on the presenter user interface if the signal indicates that the slide specific text is missing a portion of the presenter text;
- receive a positive modification selection or a negative modification selection from the presenter user interface in response to displaying the modify lecture text message on the presenter user interface; and
- add the missing portion of the presenter text to the slide specific text if the positive modification selection is received.

6. The medical system of claim 4 or 5, wherein the text comparison module is implemented using natural language processing, wherein the natural language processing preferably comprises term frequency-inverse document frequency processing.

7. The medical system of claim 4 or 5, wherein the text comparison module is implemented using a text comparison large language model.

8. The medical system of claim 1, wherein the presentation slide specific user data comprises a question text, wherein execution of the machine executable instructions further causes the computational system to determine the presentation slide specific modification text using the question text.

9. The medical system of any one of claims 2 through 8, wherein execution of the machine executable instructions further causes the computational system to:
- display the question text on a user interface; and
- receive the presentation slide specific modification text from the user interface in response to displaying the question text.

10. The medical system of any one of claims 2 through 9, wherein the memory further stores a text generating large language model (326) configured to generate the presentation slide specific modification text in response to receiving the question text and preferably receiving the presentation slide specific lecture text, wherein execution of the machine executable instructions further causes the computational system to receive (404) the presentation slide specific modification text in response to inputting the question text and preferably the presentation slide specific lecture text into the text generating large language module.

11. The medical system of any one of the preceding claims, wherein the presentation slide specific user data comprises physiological data (328), wherein execution of the machine executable instructions further causes the computational system to enable or disable (406) modification of the presentation slide specific lecture text using the physiological data.

12. The medical system of any one of the preceding claims, wherein the memory further comprises an image recognition module configured to output one or more image metric in response to receiving a video stream, wherein the presentation slide specific user data comprises the video stream comprising user specific images collected by the multiple telecommunication communication devices, wherein execution of the machine executable instructions further causes the computational system to receive the one or more image metric in response to inputting the image stream into the image recognition module, wherein execution of the machine executable instructions further causes the computational system to enable or disable modification of the presentation slide specific lecture text using the one or more image metric.

13. The medical system of any one of the preceding claims, wherein the medical system further comprises any one of the following:
- a medical imaging system (104), wherein the at least one presentation slide comprises acquired medical images and/or a control user interface rendering;
- a medical image database (104), wherein the at least one presentation slide at least partially comprises data retrieved from the medical image database; and
- combinations thereof.

14. A method of operating a medical system (100, 300), wherein the medical system comprises a communication system (110) configured for establishing a teleconference session between multiple telecommunication devices, wherein the medical system further comprises a memory (116) storing at least one presentation slide (122) and presentation slide specific lecture text (124),
Wherein the method comprises repeatedly:
- receiving (200) presentation slide specific user data (128) from the communications system;
- constructing (202) presentation slide specific modification text (126) using the presentation slide specific user data; and
- modifying (204) the presentation slide specific lecture text using the presentation slide specific modification text.

15. A computer program comprising machine executable instructions (120) for execution by a computational system (110) controlling a medical system (100, 300), wherein the medical system comprises a communication system (110) configured for establishing a teleconference session between multiple telecommunication devices (106), wherein the medical system further comprises a memory (116) storing at least one presentation slide (122) and presentation slide specific lecture text (124), wherein execution of the machine executable instructions causes the computational system to repeatedly:
- receive (200) presentation slide specific user data (128) from the communications system;
- construct (202) presentation slide specific modification text (126) using the presentation slide specific user data; and
- modify (204) the presentation slide specific lecture text using the presentation slide specific modification text.
